# EUROPEAN PATENT SPECIFICATION

(11) **EP 1 694 828 B1**
(45) Date of publication and mention of the grant of the patent: **16.03.2011**
(21) Application number: 04814038.8
(22) Date of filing: 10.12.2004
(51) Int. Cl.: C12N 5/07, C12N 7/00, C12N 5/00

(54) **SERUM-FREE VERO CELL BANKING PROCESS**
SERUMFREIES VERO-ZELLBANKEN-HERSTELLUNGSVERFAHREN
PROCEDE DE STOCKAGE DE CELLULES VERO SANS SERUM

(30) Priority: 19.12.2003 US 531379 P
(43) Date of publication of application: 30.08.2006
(73) Proprietor: Wyeth LLC, Madison, NJ 07940 (US)
(72) Inventor: ALLIKMETS, Ene, Cornwall on Hudson, NY 12520 (US); NICHOLS, Amy, Helen, Warwick, NY 10990 (US); PLUMMER, Dorothy, Jean, Florida, NY 10921 (US)
(74) Representative: Ricol, David Gerard
(86) International application number: PCT/US2004/041801
(87) International publication number: WO 2005/066332

(56) References cited:
- WO-A-01/23527
- AT-B- 409 379
- US-B1- 6 406 909

## Description

### BACKGROUND OF THE INVENTION

The present invention relates to a serum-free cell-freezing medium and the use of this medium in a process for generating stable serum-free cell banks for viral vaccine production.

Fetal bovine serum (FBS) is a cryostabilizing agent commonly used in cell banking. Serum-containing culture systems, however, are becoming undesirable for the large-scale production of vaccines. There are a number of disadvantages of serum supplementation, including batch-to-batch variation in composition, the high protein content that hinders product purification, and the potential for viral, mycoplasma, or prion contamination. Furthermore, the recent threat to human health caused by the undefined agents of bovine spongiform encephalopathy (BSE) is likely to limit the continued use of bovine serum in culture processes used for the synthesis of health care products such as viral vaccines [Butler, et al., Biotechnol. Prog., 16, 854-858 (2000)]. Therefore, a cryostabilizing agent of non-animal origin is needed to replace FBS.

### SUMMARY OF THE INVENTION

The present invention provides a serum-free cell-freezing medium consisting essentially of a virus-production serum-free medium (VP-SFM) supplemented with (a) an enzymatic hydrolysate cryostabilizer selected from the group consisting of soy hydrolysate and rice hydrolysate, added at about 2 g per liter of said medium, and (b) dimethylsulfoxide (DMSO). In one embodiment, the serum-free cell-freezing medium is supplemented with about 10% DMSO.

The present invention further provides for the use of this serum-free cell-freezing medium in a process that generates standardized and consistent master and working cell banks, which are used as raw material in vaccine production. This process comprises the steps of:
(a) initiating a culture, which comprises thawing frozen Vero cells and adding them to growth medium in a T-150 cm² flask, wherein the growth medium consists of VP-SFM with 4mM L-glutamine, incubating the cells overnight at 37°C and 5% CO₂, and refeeding the culture with fresh growth medium the following day;
(b) propagating and amplifying the cells, which comprises growing the cells to confluence in the T-150 cm² flask incubated at 37°C and 5% CO₂, removing the medium, washing the flask phosphate buffered saline (PBS) without calcium and magnesium, adding trypsin to the flask and incubating at room temperature for a sufficient time to dislodge the cells from the flask, neutralizing the trypsin with soybean trypsin inhibitor (STI) and adding VP-SFM for nutritional support, seeding the resulting suspension into five T-150 cm² flasks and adding VP-SFM to each flask to a level of 50 ml, incubating the cells at 37°C and 5% CO₂ for three to four days, pooling the cell suspensions from the five flasks, seeding a cell factory with the pooled suspension, refeeding the cell factory, and harvesting the cell factory; and
(c) freezing the cell bank, which comprises centrifuging the harvested cells from the cell factory for 10 minutes at 210xg at 4°C, resuspending the cells in the serum-free cell-freezing medium of claim 1 at a density of 2 x 10⁶ to 2 x 10⁷ cells/ml, dispensing the cell suspension into cryovials at one ml of cell suspension per vial, freezing the cells using an active rate control freezer, and storing the cells in liquid nitrogen,
   wherein the stable serum-free Vero cell bank thus produced has a cell viability of at least 80% and a recovery doubling time between 40 and 60 hours after one year.

In a specific embodiment, this process comprises the steps of:
(a) initiating a culture, which comprises thawing 2 x 10⁷ frozen Vero cells and adding them to 50 ml of growth medium in a T-150 cm² flask to obtain a cell density of 4-5x10⁵ cells/ml, wherein the growth medium consists of VP-SFM with 4mM L-glutamine, incubating the cells overnight at 37°C under 5% CO₂, and refeeding the culture with fresh growth medium the following day;
(b) propagating and amplifying the cells, which comprises growing the cells to confluence in the T-150 cm² flask incubated at 37°C under 5% CO₂, removing the medium, washing the flask two times with 20 ml phosphate buffered saline (PBS) without calcium and magnesium, adding 5 ml trypsin to the flask and incubating at room temperature for a sufficient time to dislodge the cells from the flask, neutralizing the trypsin with 5 ml of soybean trypsin inhibitor (STI) and adding 10 ml of VP-SFM for nutritional support, seeding the resulting suspension into five T-150 cm² flasks at a concentration of 4 x 10⁴ cells/cm² and adding VP-SFM to each flask to a level of 50 ml, incubating the cells at 37°C under 5% CO₂ for three to four days, pooling the cell suspensions from the five flasks, seeding a cell factory with the pooled suspension, refeeding the cell factory, and harvesting the cell factory; and
(c) freezing the cell bank, which comprises centrifuging the harvested cells from the cell factory for 10 minutes at 210xg at 4°C, resuspending the cells in the serum-free cell-freezing medium described above at a density of 1-2 x 10⁷ cells/ml, dispensing the cell suspension into cryovials vial at one ml of cell suspension per vial, freezing the cells using an active rate control freezer, and then storing the cells in liquid nitrogen,
   wherein the stable serum-free Vero cell bank thus produced has a cell viability of at least 80% and a recovery doubling time between 40 and 60 hours after one year.

The present invention further provides a stable serum-free Vero cell bank for vaccine production produced by the process immediately described above, wherein the average cell bank viability is at least 80% and the average recovery doubling time is between 40 and 60 hours after one year.

### BRIEF DESCRIPTION OF THE DRAWINGS

FIG. 1 is a scale-up scheme for 100 vial cell banks. Cell banks of 200 vials and 400 vials follow the same scheme, using two cell factories and four cell factories, respectively.
FIG. 2 is a graph comparing the cell viabilities of 10 vial serum-free cell banks containing plant-derived cryostabilizers with that of FBS-containing cell banks.
FIG. 3 is a graph comparing the recovery doubling times of 10 vial serum-free cell banks containing plant-derived cryostabilizers with that of FBS-containing cell banks.
FIG. 4 is a graph comparing the cell viabilities of large-scale cell banks (100, 200 and 400 vials), which were made using Hy-Soy® and HyPep® Rice as the cryostabilizers.
FIG. 5 is a graph comparing the recovery doubling times of large-scale cell banks (100, 200 and 400 vials), which were made using Hy-Soy and HyPep Rice as the cryostabilizers.

### DETAILED DESCRIPTION OF THE INVENTION

Implementation of a correct and standardized cell banking process is fundamental to the provision of cell stocks with consistent performance and characteristics. Cell banking should include a master cell bank of homogeneous source cells and numerous working cell banks generated from the master cell bank. The number of vials of a given bank will vary depending on the intended use, but a typical master cell bank may contain 200 vials and a typical working cell bank may contain up to 400 vials, utilizing the banking process of the present invention. This process ensures a long-term supply of standardized cells that will provide a stable substrate for consistent production.

Vero cells (immortalized African Green Monkey kidney cells, ATCC # CCI-81) have been known and used in the art for the production of human viral vaccines for many years. The cells are well characterized and have an excellent safety record.

The serum-free Vero cell banking process of the present invention consists of three major steps: initiating the culture, propagating and amplifying the cells, and freezing the bank. Optimization of each of these steps has made it possible to generate high quality serum-free cell banks. "High quality" means the viability of the cells is high and the recovery is fast, i.e., the recovery doubling time is low. The combination of these cell growth and freezing conditions results in improved recovery of Vero cells. Two of the most important recovery parameters for consistent production of cells from a cell bank are the recovery time and the thaw viability. The recovery time is closely related to the plating efficiency but it also provides more information about the performance of the cells after thawing. Both of these parameters were measured and used to optimize the instant cell banking process.

Phenol red was eliminated from the cell-freezing medium due to reports that this component could have estrogenic effects on the cells. [Berthois, et al. Cell Biology, 83, 2496-5000 (1986)]. The composition of the cell-freezing medium was optimized to contain 2 g/L Hy-Soy (or HyPep Rice), 10 % DMSO, and the remainder VP-SFM as a growth medium.

The "VP-SFM" used herein was represented by Gibco™ VP-SFM (commercially available from Invitrogen Corp., Carlsbad, CA) modified to contain the ingredients listed in Table 1.

**Table 1**

| **GIBCO VP-SFM** **(modified)** | | |
|---|---|---|
| L-Alanine | Sodium Phosphate Dibasic Anhyd | Putrescine 2HCl |
| Glycine | Magnesium Sulfate Anhyd | Recombinant Human Insulin |
| L-Arginine HCl | Calcium Chloride Anhyd | Epidermal Growth Factor (EGF), Recombinant |
| L-Asparagine H₂O | D Calcium Pantothenate | Sodium Phosphate Dibasic Anhyd. |
| L-Aspartic Acid | Magnesium Chloride Anhyd | Sodium Phosphate Monobasic H₂O |
| L-Cysteine HCl H₂O | Ascorbic Acid 2 Phosphate Magnesium Salt | HEPES |
| L-Cystine 2HCl | Sodium Chloride | Ferric Citrate Complex |
| L-Glutamic Acid | Vitamin A Acetate | Adenine Sulfate |
| Glutathione Reduced | Vitamin D₂ | Adenosine-5-Triphosphate |
| L-Histidine HCl H₂O | Menadione | D-Glucose (Dextrose) |
| L-Isoleucine | DL Lipoic Acid Thioctic | Vitamin B₁₂ |
| L-Leucine | Linoleic Acid | Choline Chloride |
| L-Lysine HCl | Uracil | I-Inositol |
| L-Methionine | Thymidine | Niacinamide |
| L-Phenylalanine | Biotin | Pyridoxal HCl |
| L-Proline | Folic Acid | 2-Deoxyribose |
| L-Serine | Riboflavin | Ethanolamine HCl |
| L-Threonine | Sodium Hypoxanthine | Phosphoethanolamine |
| L-Tryptophan | Sodium Xanthine | Potassium Chloride |
| L-Valine | Para Amino Benzoic Acid | Sodium Pyruvate |
| Thiamine HCl | Nicotinic Acid | Proprietary plant hydrolysate |
| L-Tyrosine Disodium Salt | Pyridoxine HCl | Yeast Extract UF |

As a process improvement, the culture was initiated from double the cell density that has been customary in the art. Therefore, rather than starting with one vial, the culture is initiated from two frozen vials from the source bank thawed into one T-150 cm² flask. Warm VP-SFM is added drop wise to avoid shock or damage to the cells. Refeeding the culture the next day after thawing removes remaining cryostabilizer and cell debris, and improves the recovery.

The scale-up scheme and schedule of the present invention enables the propagation of Vero cells with a minimal number of passages (e.g., no more than four passages) and manipulations, while still keeping the cell concentration consistently high to maintain a healthy culture. The scale-up scheme is: two frozen vials from the source bank thawed into one T-150 cm² flask, expanded into five T-150 cm² flasks that are used to inoculate one 10-tray cell factory. A 100-vial cell bank can be generated from one cell factory. This scheme allows for easy adjustments in bank size.

The trypsinization scheme was also optimized. Two buffer rinses of the cell monolayer were found to be optimal for good recovery after thawing. Gamma-irradiated porcine trypsin was chosen to minimize possible viral contamination. Because sufficient heat transfer to all ten levels of the cell factory is questionable, creating unnecessary microenvironments, the trypsinization is performed at room temperature with chilled trypsin.

The combination of freezing process conditions improved recovery of the bank considerably. First, the harvested and pooled cell suspension is centrifuged at 210xg for 10 minutes as opposed to 5 or 7 minutes, which causes considerable cell loss. Resuspension is performed gently with cold freezing medium. Second, dispensing the cells into cryovials with a repeater pipettor made the dispensing procedure faster and reduced the time cells had to be exposed to the freezing medium before the actual freezing cycle. And third, the cell suspension during dispensing into cryovials and the already filled vials were cooled down to 2-8°C.

Two aspects of the cryopreservation of Vero cells needed to be addressed for the banking process: the optimum cell density in the cryovial, and programming the rate control freezer for good recovery of the cells upon thawing. During the phase change, as a material freezes the heat of transition from liquid to solid is released (the heat of fusion). The program was optimized so that the heat of fusion would be accounted for, forcing the sample to freeze at a constant rate. It was clearly shown that the optimum cell density was 1-2 X 10⁷ cells/ml and that the modifications to the freezing program were contributing to the good recovery of cells.

The serum-free Vero cell banking process of the present invention provides a standardized and consistent way to generate reliable and stable cell banks for viral vaccine production. Animal-derived products were replaced in the growth and freezing media by plant-derived substances such as Hy-Soy and HyPep Rice, which serve as cryostabilizers. The results show that serum-free cell banks using either 2 g/L Hy-Soy or 2 g/L HyPep Rice are stable for at least one year.

The improvements resulting in the instant cell banking process increase the viability of the cells upon thawing and reduce recovery time, which allows a more precise schedule for manufacturing and more consistent processes. It is also now possible for the manufacturing process to start from a smaller number of frozen vials. Therefore one full-size 400-vial bank will last longer and consecutive runs will be more consistent.

Having now generally described the invention, the same will be more readily understood through reference to the following examples that are provided by way of illustration, and are not intended to be limiting of the present invention, unless specified.

### EXAMPLES

Several agents were tested as cryostabilizers in this study. Cell banks containing Hy-Soy, HyPep Rice, HyPep Wheat and methylcellulose were compared with FBS-containing cell banks, and cell bank stability was examined. It was demonstrated that Hy-Soy and HyPep Rice were as effective as FBS as cryostabilizers for the first year of the study. Therefore, Hy-Soy can be used in serum-free cell banks and HyPep Rice can be used as an alternative.

### Materials and Methods

*Personal Protective Equipment:* Sterile gloves, sterile sleeves, sterile jumpsuit, clean room head covers, clean room beard covers, clean room foot covers.

*Disposable Equipment:* Disposable serological pipettes; 1.8 ml Sarstedt cryovials; Corning Costar 75 cm² and 150 cm² tissue culture flasks; ten tray Nunc cell factories [www.nuncbrand.com].

*Glassware and Accessories:* Glass bell with C-flex tubing for dispensing medium from 10 L and 20 L medium totes; Gelman filters with C-flex tubing; cell factory adapters; 500 ml, 1 L, 2 L and 5 L bottles with transfer apparatus and tubing; hand bulb.

*Reagents:* Gibco™ VP-SFM formulated to contain the ingredients in Table 1; Gibco soybean trypsin inhibitor (STI); Gibco L-glutamine; Gibco phosphate buffered saline (PBS) without calcium and magnesium; Gibco bovine serum albumin (BSA); Hy-Soy, HyPep Rice, and HyPep Wheat from Quest International (Norwich, NY); Sigma (St. Louis, MO) dimethylsulfoxide (DMSO) and methylcellulose; 70% isopropyl alcohol; Gibco 0.25% irradiated trypsin/citrate; Gibco 0.025 M sodium citrate buffer, Hyclone (Logan, UT) Fetal Bovine Serum (FBS) or irradiated FBS (irFBS); Gibco Dulbecco's Minimal Essential Medium (DMEM); water for injection or deionized water; Gibco 0.05% Trypan Blue Solution.

*Equipment:* Bio safety hood; laminar flow hood or bio safety cabinet; spinner flask base; automatic (repeator) pipettor; microscope; inverted microscope; hemacytometer; circulating water bath; Forma Cryomed rate control freezer (RCF); peristaltic pump; dosing pump; Forma incubator at 37°C and 5% CO₂; Forma centrifuge; 216 swinging bucket type rotor.

### Example 1

### Thawing and Cell Growth

Two frozen stock vials containing 2x10⁷ Vero cells were placed in a 37°C water bath. The vials were held in the water bath with agitation, and checked frequently until frozen cells were thawed. The outside of the vials were sanitized with 70% isopropyl alcohol and placed in a laminar flow hood. Thawed cells were quickly transferred to a T-150 cm² flask and 50 ml of growth medium was added drop wise while rocking the culture container. Growth medium consisted of VP-SFM with 4 mM L-glutamine. After approximately half of the medium was added, the rest was added at a slightly faster rate. The cells were placed in a Forma incubator (37°C, 5%CO₂) and allowed to attach overnight. The medium was aspirated off and 50 ml of fresh growth medium was added to the flask the following day.

### Example 2

### Cell Passing

Cells were grown to confluence (4 or 5 days) in the T-150 cm² flask incubated at 37°C and 5% CO₂ in the Forma incubator. The medium was aspirated off and the flask was washed two times with 20 ml PBS, without magnesium and calcium. Five ml of trypsin was added to the flask and the flask was incubated at room temperature for 2 to 3 minutes to remove the cells from the flask. The trypsin was neutralized with 5 ml of STI and 10 ml of VP-SFM was added for nutritional support until the procedure was completed. The cells, now in a suspension, were sampled for counting. One ml of Trypan-blue solution was added to a one ml cell sample. This was mixed gently with a vortexor. Ten µl of mixture was placed in the chamber of a hemacytometer. Cells resisting the stain were counted as live and the total cell count was determined.

The cell suspension was then seeded into five new T-150 cm² flasks at a concentration of 4 x10⁴ cells/cm². VP-SFM was added to each T-150 cm² flask to a level of 50 ml, and then the flasks were placed in the Forma incubator at 37°C and 5% CO₂. Three days later the flasks were refed. The flasks were incubated for a total of five days. The cell suspensions from the five flasks were pooled, and the pooled suspension was used to seed a Nunc cell factory. One confluent cell factory produces enough cells for a 100-vial cell bank.

### Example 3

### Cell Factory Set-up and Seeding

One of the adapters provided by Nunc for the cell factory was connected to a 10-inch piece of silicone tubing (ID 3/16", OD 3/8"). A tubing connector was used to attach an 8-inch length of C-flex tubing (ID .125", OD .200") to the silicone tubing connected to the cell factory. This is the inlet/outlet adapter. Both ends of tubing were wrapped with bioshield. The second adapter provided by Nunc for the cell factory was attached to a three-inch piece of silicone tubing (ID 3/16", OD 3/8") with a small Gelman filter (0.22 µm). The adapter end was wrapped with bioshield. Both adapters were autoclaved for 30 minutes with a 15-minute dry time. In the BioSafety Cabinet (BSC), the adapters were attached to the cell factory as per the instruction manual provided by Nunc. Either port on the cell factory may be used for the inlet/outlet line and filter assembly line. A 10-L bag of VP-SFM or 3 x 1 L bottles were obtained from the chill room and warmed to 37°C. Five L C-flex transfer lines were placed in sterile 5-L bottle for cell seeding. All bags used were equipped with C-flex by the manufacturer. The Sterile Connection Device (SCD) was used to connect the medium to the seeding bottle and 2.5 L of medium was dispensed into the seeding bottle. Cell suspension was added to the seeding bottle. The SCD was used to connect the seeding bottle to the cell factory. The cell factory was placed in the fill and drain position. The medium with cells was slowly hand pumped into the cell factory. The silicon tubing connected to the inlet/outlet line was immediately clamped off. A 0.22 µm Gelman filter (Acro 50) with C-flex was connected to the cell factory using the SCD. The cell factory was placed in the level check position. When liquid was evenly distributed into the ten levels, the cell factory was placed into the horizontal position, and placed in a 37°C, 5% CO₂ incubator.

### Example 4

### Cell Factory Refeed

The cell factory was refed on days 2 and 5 after seeding. Using the SCD, the void bag was connected to the cell factory. The cell factory was gently placed in the fill and drain position and liquid level marked. The void bag was placed on the floor to help drain the medium. It was necessary at times to pressurize the cell factory. A hand bulb was connected to the filter assembly to gently pressurize the cell factory. Once flow began, the hand bulb was disconnected. When medium was emptied, the bag containing the refeed medium was connected to the cell factory using the SCD and the same volume of medium was slowly hand pumped into the cell factory. The cell factory was placed in the level check position. After level check was completed the cell factory was placed in its horizontal working position in the 37°C, 5% CO₂ incubator.

### Example 5

### Cell Factory Harvest

The cell factory was drained as described above. The first wash consisted of either 300-ml of 25mM sodium citrate buffer or 300 ml PBS without calcium or magnesium. The first wash was pumped into the cell factory. The wash was allowed to equalize in each level. The cell factory was shaken gently to rinse all the cell surfaces. The first wash was drained and a second wash was applied in the same manner as the first wash. The second wash was drained and a bottle of trypsin solution (1.4 ml of Gibco 2.5% trypsin in 300 ml of 2.5 mM sodium citrate buffer) was attached to the cell factory. The trypsin solution was pumped into the cell factory and the cell factory was gently rocked so that all the cell surfaces were covered. The cell surface was examined every 2 or 3 minutes to see if the cells had begun to detach. When cells began to detach from the cell factory surface, the cell factory was rigorously tapped to release the rest of the cells. This was done until the cell factory appeared clear. The cell factory was then connected to a cell collection bottle containing 100 ml of 5 mg/ml soybean trypsin inhibitor (STI). The cell mixture was drained into this bottle. The medium wash bottle was connected to the cell factory, care being taken to clamp the cell collection bottle. The bottle containing 600 ml of growth medium was attached to the cell factory to rinse the surfaces. The cell factory was reconnected to the cell collection bottle and the rinse medium was drained. This wash can contain up to 1/3 of the total cells.

### Example 6

### Freezing Protocol

Cells harvested from flasks or Nunc cell factories were sampled for counting, pooled, and centrifuged for 10 minutes at 210xg at 4°C in the Forma centrifuge. Cell-freezing medium was prepared consisting essentially of VP-SFM, supplemented with 10% DMSO, and 2 g/L of one of the cryostabilizers tested in this study. The cells were kept on ice during the process. The cells were then resuspended in the freezing medium at a density of 1.5 x 10⁷ cells/ml. The cell concentrate was put into 1.8 ml Sarstedt cryovials at one ml of cell suspension per vial. Cells were frozen using an active rate control freezer. After freezing, the cells were stored in liquid nitrogen.

### Example 7

### Cell Banks

*Cell banks:* Initial experiments examining various cryostabilizers were accomplished using 10 vial cell banks derived from T-flasks. Based on the results of the 10 vial cell banks, the promising cryostabilizers were used in larger cell banks of 100, 200 and 400 vials. The cells needed for large-scale cell banks were grown in cell factories.

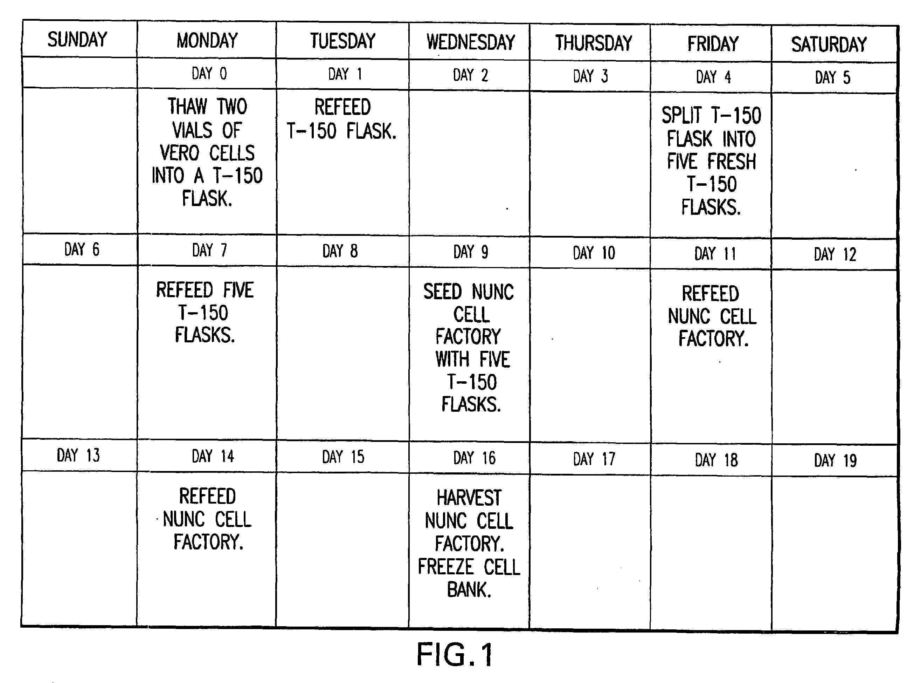

*Cell expansion for large-scale cell banks:* The expansion of cells for cell banks was performed as follows. Two vials of frozen cells were thawed for each 100 vials of the cell bank to be made. Two vials were used to inoculate one T-150 cm² flask. The T-150 cm² flask was refed the following day. After 4 days growth, the cells were split into five T-150 cm² flasks. Three days after the split, the flasks were refed. The flasks were incubated for five days. The flasks were harvested and used to inoculate one Nunc 10 layer cell factory. The cell factory was refed after two and five days. On the seventh day, the cell factory was harvested. See FIG. 1 for the schedule of steps used to prepare the cell banks.

*Cell bank testing:* Five vials from each cell bank were thawed as described in Example 1. Each vial was used to seed two T-75 cm² flasks containing 25 ml of VP-SFM. Cells were harvested from flasks when they were close to confluence and the following day. Doubling times were determined using cell count and time of growth. Cell viability was determined with Trypan-blue as described above.

### RESULTS AND DISCUSSION

*Cell Bank Recovery:* Two parameters were used to determine cell bank stability: cell recovery doubling time (flasks harvested and counted when they were close to confluence and the following day) and the viability of cells after thawing. Cells were thawed immediately after freezing and at various time points. The cells were immediately tested for viability and placed in T-flasks. The T-flasks were harvested to determine cell recovery doubling times.

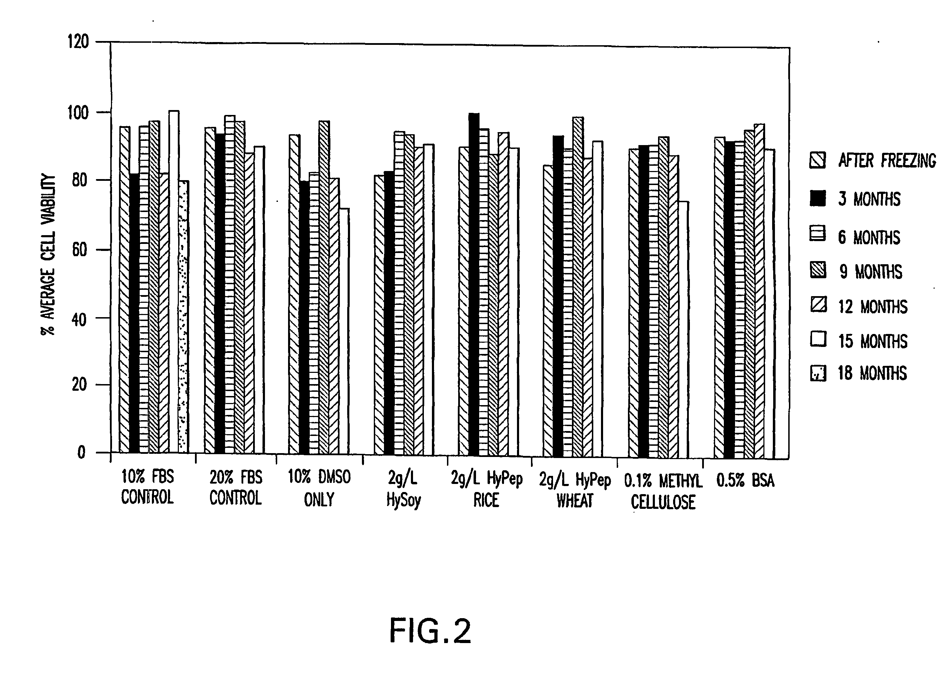

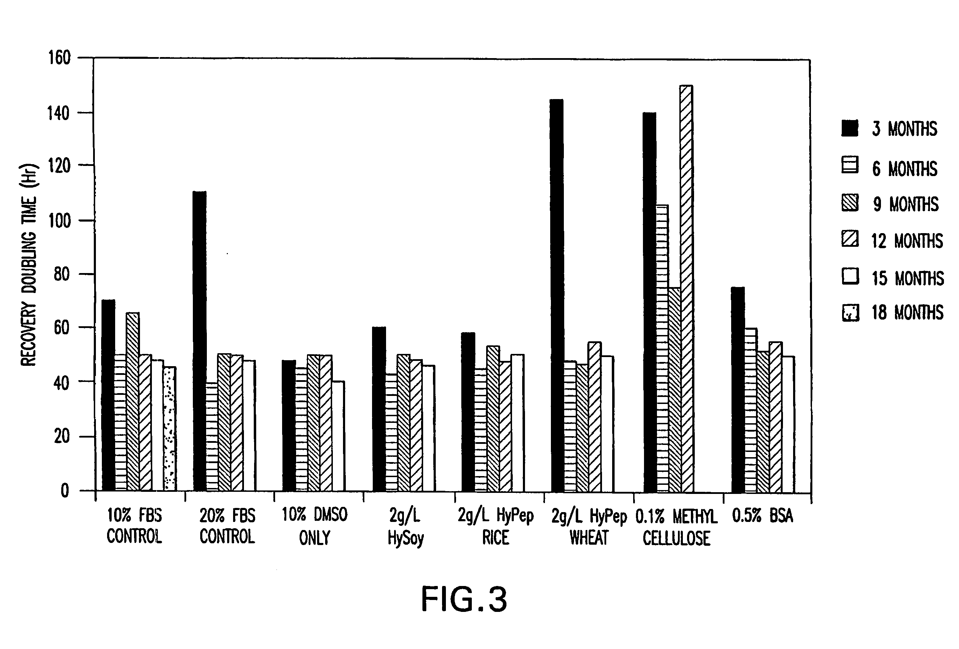

*Ten Vial Vero Cell Bank* (See FIGS. 2 and 3): In Figure 2 the average of five samples of each cell bank was taken at various time points. The samples were quickly thawed and viability was determined using Trypan-blue. In Figure 3 the average of five samples of each cell bank was taken at various time points. The samples were quickly thawed and placed on T flasks. Cells were counted periodically to determine recovery-doubling times. All cell banks had greater than 90% viability after being stored in liquid nitrogen for one year. The 10% DMSO only cell bank and the 0.1 % methylcellulose cell bank dropped to below 80% at 15 months. It was also seen that the recovery of the methylcellulose bank was very poor with doubling times of greater than 100 hours as compared to 45 to 50 hour doubling times of the other cell banks. The Hy-Soy, HyPep Rice and HyPep Wheat all had the doubling times and viabilities that were seen in the control cell banks (FBS- containing banks). Based on these data, large-scale cell banks of 100, 200 and 400 vials were made using Hy-Soy and HyPep Rice as cryostabilizers.

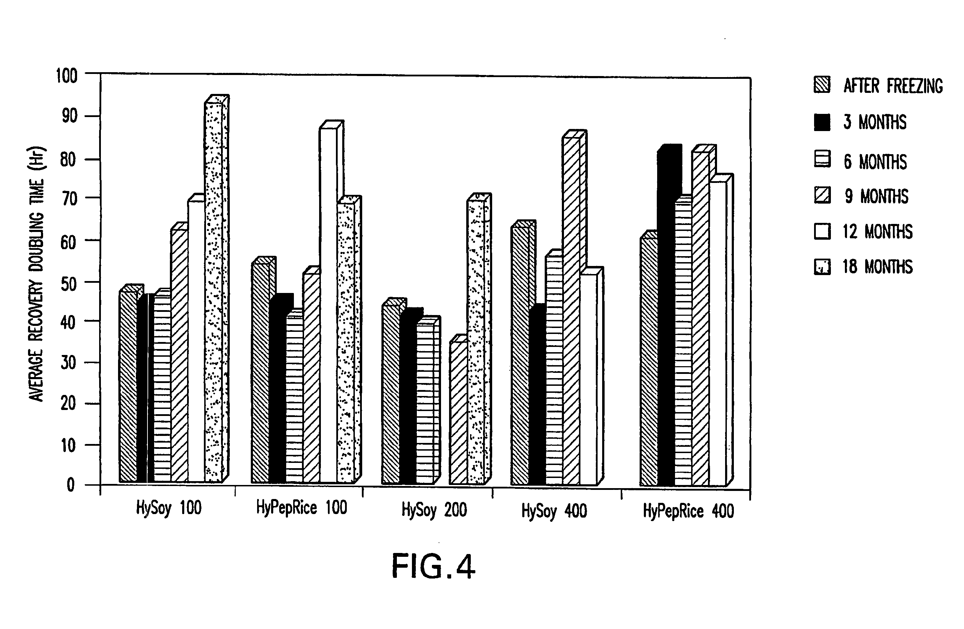

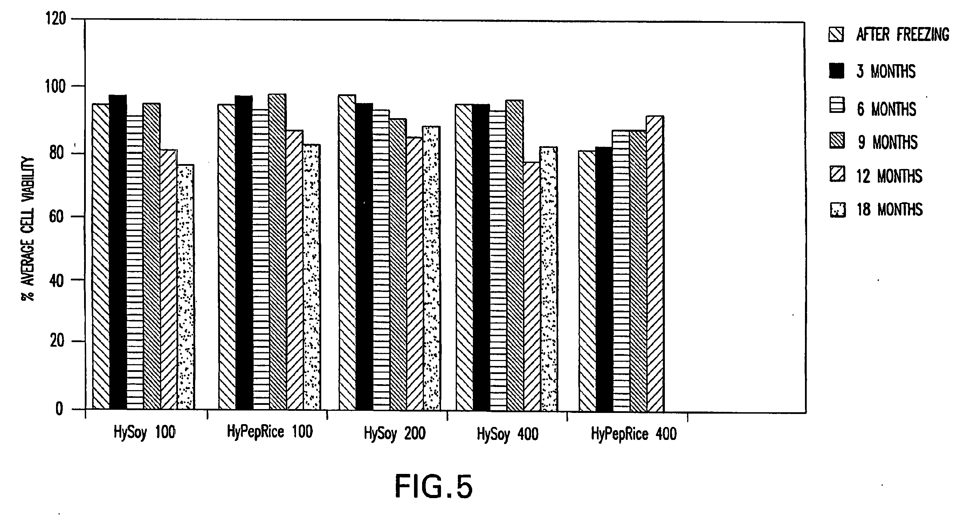

*Large-Scale Cell Banks* (See FIGS. 4 and 5): In Figure 4 the average of five samples of each cell bank was taken at various time points. The samples were quickly thawed and placed on T flasks. Cells were counted periodically to determine recovery-doubling times. In Figure 5 the average of five samples of each cell bank was taken at various time points. The samples were quickly thawed and viability was determined using Trypan-blue. The one hundred vial Hy-Soy and HyPep Rice-containing cell banks' viability remained greater than 80% for nine months. Cell recovery also remained consistently between 45 to 60 hours doubling time. These results are consistent with the serum-containing cell banks (see FIGS. 2 and 3). The 200 vial Hy-Soy bank and the 400 vial Hy-Soy bank also showed a viability of over 80% and recovery doubling times between 40 hours and 60 hours over a twelve month period. The Hy-Soy and HyPep Rice large-scale cell banks are all comparable to the FBS-containing cell banks. From the above experimental results, it can be concluded that serum-free cell banks using either 2 g/L Hy-Soy or 2 g/L HyPep Rice are stable for up to one year.

This invention has been described by a direct description and by examples. As noted above, the examples are meant to be only examples and not to limit the invention in any meaningful way. Additionally, one having ordinary skill in the art to which this invention pertains in reviewing the specification and claims that follow, would appreciate that there are equivalents to those claimed aspects of the invention. The inventors intend to encompass those equivalents within the reasonable scope of the claimed invention.

## Claims

1. A serum-free cell-freezing medium consisting essentially of a virus-production serum-free medium (VP-SFM) supplemented with (a) an enzymatic hydrolysate cryostabilizer selected from the group consisting of soy hydrolysate and rice hydrolysate, added at about 2 g per liter of said medium, and (b) dimethylsulfoxide (DMSO).

2. The serum-free cell-freezing medium of claim 1, which is supplemented with about 10% DMSO.

3. A process for generating a stable serum-free Vero cell bank, which process comprises the steps of:
(a) initiating a culture, which comprises thawing frozen Vero cells and adding them to growth medium in a T-150 cm² flask, wherein the growth medium consists of VP-SFM with 4mM L-glutamine, incubating the cells overnight at about 37°C and 5% CO₂, and refeeding the culture with fresh growth medium the following day;
(b) propagating and amplifying the cells, which comprises growing the cells to confluence in the T-150 cm² flask incubated at about 37°C and 5% CO₂, removing the medium, washing the flask with phosphate buffered saline (PBS) without calcium and magnesium, adding trypsin to the flask and incubating at room temperature for a sufficient time to dislodge the cells from the flask, neutralizing the trypsin with soybean trypsin inhibitor (STI) and adding VP-SFM for nutritional support, seeding the resulting suspension into five T-150 cm² flasks and adding VP-SFM to each flask to a level of 50 ml, incubating the cells at about 37°C and 5% CO₂ for three to four days, pooling the cell suspensions from the five flasks, seeding a cell factory with the pooled suspension, refeeding the cell factory, and harvesting the cell factory; and
(c) freezing the cell bank, which comprises centrifuging the harvested cells from the cell factory for 10 minutes at 210xg at 4°C, resuspending the cells in the serum-free cell-freezing medium of claim 1 at a density of 2 x 10⁶ to 2 x 10⁷ cells/ml, dispensing the cell suspension into cryovials at one ml of cell suspension per vial, freezing the cells using an active rate control freezer, and storing the cells in liquid nitrogen,
wherein the stable serum-free Vero cell bank thus produced has a cell viability of at least 80% and a recovery doubling time between 40 and 60 hours after one year.

4. A process for generating a stable serum-free Vero cell bank according to claim 3, which process comprises the steps of:
(a) initiating a culture, which comprises thawing 2 x 10⁷ frozen Vero cells and adding them to 50 ml of growth medium in a T-150 cm² flask to obtain a cell density of 4-5x10⁵ cells/ml, wherein the growth medium consists of VP-SFM with 4mM L-glutamin, incubating the cells overnight at 37°C and 5% CO₂, and refeeding the culture with fresh growth medium the following day;
(b) propagating and amplifying the cells, which comprises growing the cells to confluence in the T-150 cm² flask incubated at 37°C and 5% CO₂, removing the medium, washing the flask two times with 20 ml phosphate buffered saline (PBS) without calcium and magnesium, adding 5 ml trypsin to the flask and incubating at room temperature for a sufficient time to dislodge the cells from the flask, neutralizing the trypsin with 5 ml of soybean trypsin inhibitor (STI) and adding 10 ml modified VP-SFM for nutritional support, seeding the resulting suspension into five T-150 cm² flasks at a concentration of 4 x 10⁴ cells/cm² and adding VP-SFM to each flask to a level of 50 ml, incubating the cells at 37°C and 5% CO₂ for three to four days, pooling the cell suspensions from the five flasks, seeding a cell factory with the pooled suspension, refeeding the cell factory, and harvesting the cell factory; and
(c) freezing the cell bank, which comprises centrifuging the harvested cells from the cell factory at for 10 minutes at 210xg at 4°C, resuspending the cells in the serum-free cell-freezing medium of claim 2 at a density of 1-2 x 10⁷ cells/ml, dispensing the cell suspension into cryovials at one ml of cell suspension per vial, freezing the cells using an active rate control freezer, and storing the cells in liquid nitrogen, wherein the stable serum-free Vero cell bank thus produced has a cell viability of at least 80% and a recovery doubling time between 40 and 60 hours after one year.

5. The serum-free cell-freezing medium of claim 1 or 2, wherein the enzymatic hydrolysate cryostabilizer is a soy hydrolysate.

6. The serum-free cell-freezing medium of claim 1 or 2, wherein the enzymatic hydrolysate cryostabilizer is a rice hydrolysate.

7. A Stable Vero cell bank having a cell viability of at least 80% and a recovery doubling time between 40 and 60 hours after one year of freezing, wherein the cell bank is free of animal-derived products, and wherein the cell bank is made using an enzymatic hydrolysate cryostabilizer comprising a soy hydrolysate or a rice hydrolysate.

## Patentansprüche

1. Serumfreies Zellgefriermedium, im Wesentlichen bestehend aus einem serumfreien Virusproduktionsmedium (virus-production serum-free medium (VP-SFM)), supplementiert mit
(a) einem enzymatischen Hydrolysat-Kryostabilisator, der aus der Gruppe, bestehend aus Sojahydrolysat und Reishydrolysat, ausgewählt ist, der mit etwa 2 g pro Liter des Mediums zugesetzt ist, und (b) Dimethylsulfoxid (DMSO).

2. Serumfreies Zellgefriermedium nach Anspruch 1, das mit etwa 10% DMSO supplementiert ist.

3. Verfahren zur Herstellung einer stabilen serumfreien Vero-Zellbank, wobei das Verfahren die Schritte:
(a) Initiieren einer Kultur, was Auftauen gefrorener Vero-Zellen und Zugeben dieser zu Wachstumsmedium in einem T-150 cm²-Kolben, wobei das Wachstumsmedium aus VP-SFM mit 4 mM L-Glutamin besteht, Inkubieren der Zellen über Nacht bei etwa 37°C und 5% CO₂ und erneutes Füttern der Kultur mit frischem Wachstumsmedium am folgenden Tag umfasst;
(b) Vermehren und Amplifizieren der Zellen, was wachsen lassen der Zellen zu Konfluenz in dem T-150 cm²-Kolben, der bei etwa 37°C und 5% CO₂ inkubiert ist, Entfernen des Mediums, Waschen des Kolbens mit Phosphat-gepufferter Salzlösung (PBS) ohne Calcium und Magnesium, Zugeben von Trypsin in den Kolben und Inkubieren bei Raumtemperatur für eine genügende Zeit, um die Zellen vom Kolben abzulösen, Neutralisieren des Trypsins mit Sojabohnen-Trypsin-inhibitor (STI) und Zusetzen von VP-SFM zur Ernährungsunterstützung, Aussähen der resultierenden Suspension in fünf T-150 cm²-Kolben und Zugeben von VP-SFM in jeden Kolben bis zu einem Level von 50 ml, Inkubieren der Zellen bei etwa 37°C und 5% CO₂ für drei bis vier Tage, Sammeln der Zellsuspensionen aus den 5 Kolben, Aussähen einer Cell Factory mit der gesammelten Suspension, erneutes Füttern der Cell Factory und Ernten der Cell Factory umfasst; und
(c) Gefrieren der Zellbank, was Zentrifugieren der geernteten Zellen aus der Cell Factory für 10 Minuten mit 210xg bei 4°C, Resuspendieren der Zellen in dem serumfreien Zellgefriermedium nach Anspruch 1 in einer Dichte von 2 x 10⁶ bis 2 x 10⁷ Zellen/ml, Verteilen der Zellsuspension in Kryophiolen mit einem ml Zellsuspension pro Phiole, Gefrieren der Zellen unter Verwendung eines Gefriergerätes mit aktiver Ratenkontrolle und Lagern der Zellen in flüssigem Stickstoff umfasst, wobei die so produzierte stabile serumfreie Vero-Zellbank, eine Zelllebensfähigkeit von wenigstens 80% und eine Verdopplungszeit nach Wiederherstellung zwischen 40 und 60 Stunden nach einem Jahr hat,
umfasst.

4. Verfahren zur Herstellung einer stabilen serumfreien Vero-Zellbank nach Anspruch 3, wobei das Verfahren die Schritte:
(a) Initiieren einer Kultur, was Auftauen von 2 x 10⁷ gefrorenen Vero-Zellen und Zugeben dieser zu 50 ml Wachstumsmedium in einem T-150 cm²-Kolben unter Erhalt einer Zelldichte von 4-5 x 10⁵ Zellen/ml, wobei das Wachstumsmedium aus VP-SFM mit 4 mM L-Glutamin besteht, Inkubieren der Zellen über Nacht bei 37°C und 5% CO₂ und erneutes Füttern der Kultur mit frischem Wachstumsmedium am folgenden Tag umfasst;
(b) Vermehren und Amplifizieren der Zellen, was wachsen lassen der Zellen zu Konfluenz in dem T-150 cm²-Kolben, der bei 37°C und 5% CO₂ inkubiert ist, Entfernen des Mediums, Waschen des Kolbens zweimal mit 20 ml Phosphat-gepufferter Salzlösung (PBS) ohne Calcium und Magnesium, Zugeben von 5 ml Trypsin in den Kolben und Inkubieren bei Raumtemperatur für eine ausreichende Zeit, um die Zellen vom Kolben abzulösen, Neutralisieren des Trypsins mit 5 ml Soja-Trypsin-Inhibitor (STI) und Zugeben von 10 ml modifiziertem VP-SFM zur Ernährungsunterstützung, Aussähen der resultierenden Suspension in fünf T-150 cm²-Kolben in einer Konzentration von 4 x 10⁴ Zellen/cm² und Zugeben von VP-SFM zu jedem Kolben zu einem Level von 50 ml, Inkubieren der Zellen bei 37°C und 5% CO₂ für drei bis vier Tage, Sammeln der Zellsuspensionen aus den 5 Kolben, Aussähen einer Cell Factory mit der gesammelten Suspension, erneutes Füttern der Cell Factory und Ernten der Cell Factory umfasst, und
(c) Gefrieren der Zellbank, was Zentrifugieren der geernteten Zellen aus der Cell Factory für 10 Minuten mit 210xg bei 4°C, Resuspendieren der Zellen in dem serumfreien Zellgefriermedium nach Anspruch 2 in einer Dichte von 1-2 x 10⁷ Zellen/ml, Verteilen der Zellsuspension in Kryophiolen mit einem ml Zellsuspension pro Phiole, Gefrieren der Zellen unter Verwendung eines Gefriergerätes mit aktiver Ratenkontrolle und Lagern der Zellen in flüssigem Stickstoff, wobei die so produzierte stabile serumfreie Vero-Zellbank, eine Zelllebensfähigkeit von wenigstens 80% und eine Verdopplungszeit nach Wiederherstellung von zwischen 40 und 60 Stunden nach einem Jahr hat,
umfasst.

5. Serumfreies Zellgefriermedium nach Anspruch 1 oder 2, wobei der enzymatische Hydrolysat-Kryostabilisator ein Sojahydrolysat ist.

6. Serumfreies Zellgefriermedium nach Anspruch 1 oder 2, wobei der enzymatische Hydrolysat-Kryostabilisator ein Reishydrolysat ist.

7. Stabile Vero-Zellbank, die eine Zelllebensfähigkeit von wenigstens 80% und eine Verdopplungszeit nach Wiederherstellung von zwischen 40 und 60 Stunden nach einem Jahr Gefrieren hat, wobei die Zellbank frei von von einem Tier stammenden Produkten ist und wobei die Zellbank unter Verwendung eines enzymatischen Hydrolysat-Kryostabilisators hergestellt wurde, der ein Sojahydrolysat oder ein Reishydrolysat umfasst.

## Revendications

1. Milieu de congélation de cellules, sans sérum, consistant essentiellement en un milieu sans sérum pour production de virus (VP-SFM) additionné (a) d'un cryostabilisant du type hydrolysat enzymatique choisi dans le groupe consistant en un hydrolysat de soja, et un hydrolysat de riz, ajouté à une quantité d'environ 2 g par litre dudit milieu, et (b) de diméthylsulfoxyde (DMSO).

2. Milieu de congélation de cellules, sans sérum, suivant la revendication 1, qui est additionné d'environ 10 % de DMSO.

3. Procédé pour engendrer une banque de cellules Vero sans sérum, stable, procédé qui comprend les étapes consistant :
(a) à déclencher une culture, opération qui comprend la décongélation de cellules Vero congelées et l'addition de ces cellules à un milieu de croissance dans une fiole T de 150 cm², le milieu de croissance consistant en milieu VM-SFM avec 4 mM de L-glutamine, la mise en incubation des cellules pendant une nuit à environ 37°C en présence de 5 % de CO₂ et la réalimentation de la culture avec du milieu de croissance frais le jour suivant ;
(b) à multiplier et amplifier les cellules, opération qui comprend la croissance des cellules jusqu'à la confluence dans la fiole T de 150 cm² en incubation à environ 37°C en présence de 5 % de CO₂, l'élimination du milieu, le lavage de la fiole avec du sérum physiologique tamponné avec un phosphate (PBS) sans calcium et magnésium, l'introduction de trypsine dans la fiole et la mise en incubation à température ambiante pendant une période de temps suffisante pour déloger la cellule de la fiole, la neutralisation de la trypsine avec l'inhibiteur de trypsine de soja (STI) et l'addition du VP-SFM à titre de support nutritionnel, l'ensemencement de cinq fioles T-150 cm² avec la suspension résultante et l'addition du VP-SFM dans chaque fiole en un volume de 50 ml, la mise en incubation des cellules à environ 37°C en présence de 5 % de CO₂ pendant trois à quatre jours, la réunion des suspensions cellulaires des cinq fioles, l'ensemencement d'une fabrique de cellules avec la suspension totale, la réalimentation de la fabrique de cellules et le recueil de la fabrique de cellules ; et
(c) à congeler la banque de cellules, opération qui comprend la centrifugation des cellules recueillies provenant de la fabrique de cellules pendant 10 minutes à 210 xg à 4°C, la remise en suspension des cellules dans le milieu de congélation de cellules, sans sérum, d'une revendication 1 à une densité de 2 x 10⁶ à 2 x 10⁷ cellules/ml, la distribution de la suspension de cellules dans des flacons de cryocongélation à raison de un ml de suspension de cellules par flacon, la congélation des cellules en utilisant un congélateur à commande de vitesse active et le stockage des cellules dans l'azote liquide,
dans lequel la banque de cellules Vero sans sérum, stable, ainsi produite, a une viabilité cellulaire d'au moins 80 % et un temps de doublement de récupération de 40 à 60 heures au bout d'un an.

4. Procédé pour engendrer une banque de cellules Vero sans sérum, stable, suivant la revendication 3, procédé qui comprend les étapes consistant :
(a) à déclencher une culture, opération qui comprend la décongélation de 2 x 10⁷ cellules Vero congelées et l'addition de ces cellules à 50 ml de milieu de croissance dans une fiole T-150 cm² pour parvenir à une densité cellulaire de 4-5 x 10⁵ cellules/ml, ledit milieu de croissance consistant en un milieu VP-SFM avec 4 mM de L-glutamine, la mise en incubation des cellules pendant une nuit à 37°C en présence de 5 % de CO₂ et la réalimentation de la culture avec ledit milieu de croissance frais le jour suivant ;
(b) à multiplier et amplifier les cellules, opération qui comprend la croissance des cellules jusqu'à confluence de la fiole T-150 cm² en incubation à 37°C en présence de 5 % de CO₂, l'élimination du milieu, le lavage de la fiole deux fois avec 20 ml de sérum physiologique tamponné avec un phosphate (PBS) sans calcium et magnésium, l'introduction de 5 ml de trypsine dans la fiole et la mise en incubation à température ambiante pendant une période de temps suffisante pour déloger les cellules de la fiole, la neutralisation de la trypsine avec 5 ml d'inhibiteur de trypsine de soja (STI) et l'addition de 10 ml de VP-SFM modifié à titre de support nutritionnel, l'ensemencement de cinq fioles T de 150 cm² avec la suspension résultante à une concentration de 4 x 10⁴ cellules/cm² et l'introduction de VP-SFM dans chaque fiole en un volume de 50 ml, la mise en incubation des cellules à 37°C en présence de 5 % de CO₂ pendant trois à quatre jours, la réunion des suspensions cellulaires provenant des cinq fioles, l'ensemencement d'une fabrique de cellules avec la suspension totale, la réalimentation de la fabrique de cellules et la collecte de la fabrique de cellules ; et
(c) à congeler la banque de cellules, opération qui comprend la centrifugation des cellules recueillies provenant de la fabrique de cellules pendant 10 minutes à 210xg à 4°C, la remise en suspension des cellules dans le milieu de congélation de cellules sans sérum de la revendication 2 à une densité de 1-2 x 10⁷ cellules/ml, la distribution de la suspension cellulaire dans des flacons de cryocongélation à raison de un ml de suspension cellulaire par flacon, la congélation des cellules en utilisant un congélateur à commande de vitesse active et le stockage des cellules dans l'azote liquide, dans lequel la banque de cellules Vero sans sérum, stable, ainsi produite a une viabilité cellulaire d'au moins 80 % et un temps de doublement de récupération de 40 à 60 heures au bout d'un an.

5. Milieu de congélation de cellules sans sérum suivant la revendication 1 ou 2, dans lequel le cryostabilisant du type hydrolysat enzymatique est un hydrolysat de soja.

6. Milieu de congélation de cellules sans sérum suivant la revendication 1 ou 2, dans lequel le cryostabilisant du type hydrolysat enzymatique est un hydrolysat de riz.

7. Banque de cellules Vero stables ayant une viabilité cellulaire d'au moins 80 % et un temps de doublement de récupération de 40 à 60 heures au bout d'un an de congélation, ladite banque de cellules étant dépourvue de produits d'origine animale, et ladite banque de cellules étant préparée en utilisant un cryostabilisant du type hydrolysat enzymatique comprenant un hydrolysat de soja ou un hydrolysat de riz.
